(19) Europäisches Patentamt
European Patent Office
Office européen des brevets

(11) **EP 3 807 421 B1**

(12) # EUROPEAN PATENT SPECIFICATION

(45) Date of publication and mention of the grant of the patent:
**13.09.2023 Bulletin 2023/37**

(21) Application number: **19728456.5**

(22) Date of filing: **07.06.2019**

(51) International Patent Classification (IPC):
*C12Q 1/6883* (2018.01)     *C12Q 1/689* (2018.01)

(52) Cooperative Patent Classification (CPC):
**C12Q 1/6883; C12Q 1/689;** C12Q 2600/158;
C12Q 2600/16

(86) International application number:
**PCT/EP2019/064949**

(87) International publication number:
**WO 2019/238561 (19.12.2019 Gazette 2019/51)**

(54) **METHOD FOR EARLY DETECTION OF A NECROTIC ENTERITIS OUTBREAK IN AN AVIAN POPULATION**

VERFAHREN ZUR FRÜHZEITIGEN ERKENNUNG EINES NEKROTISCHEN ENTERITISAUSBRUCHS BEI EINER VOGELPOPULATION

PROCÉDÉ DE DÉTECTION PRÉCOCE D'UNE ÉPIDÉMIE DE L'ENTÉRITE NÉCROTIQUE DANS UNE POPULATION AVIAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2018 US 201862685443 P**
**26.06.2018 EP 18179891**

(43) Date of publication of application:
**21.04.2021 Bulletin 2021/16**

(73) Proprietor: **Evonik Operations GmbH**
**45128 Essen (DE)**

(72) Inventors:
• IGWE, Emeka Ignatius
  80333 München (DE)
• BÖHL, Florian
  60151 Neckargemünd (DE)
• KAPPEL, Andreas
  61479 Glashütten (DE)
• THIEMANN, Frank
  48031 Nottuln (DE)
• WEISSMANN, Michaela
  32130 Enger (DE)
• WICKER, David L.
  Gainesville, Georgia 30506 (US)
• MARTIN, Ken
  Toccoa, Georgia 30577 (US)
• MIDDLEBROOKS, Casey
  Carnesville, Georgia 30521 (US)
• TILLEY, Sarah
  Demorest, Georgia 30535 (US)
• SMITH, Janet
  Mount Airy, Georgia 30563 (US)

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
WO-A1-2018/206690     WO-A2-2016/142146
CN-A- 107 090 518

• MCCOURT M T ET AL: "Sandwich ELISA detection of Clostridium perfringens cells and @a-toxin from field cases of necrotic enteritis of poultry", VETERINARY MICROBIOLOGY, ELSEVIER BV, NL, vol. 106, no. 3-4, 10 April 2005 (2005-04-10), pages 259-264, XP027620380, ISSN: 0378-1135 [retrieved on 2005-04-10]

- **MERETE HOFSHAGEN ET AL: "Toxin Production by Clostridium perfringens Isolated from Broiler Chickens and Capercaillies (Tetrao urogallus) with and without Necrotizing Enteritis", AVIAN DISEASES., vol. 36, no. 4, 1 October 1992 (1992-10-01), page 837, XP055513637, US ISSN: 0005-2086, DOI: 10.2307/1591541**
- **LEE SUNG HYEN ET AL: "Immune and anti-oxidant effects of in ovoselenium proteinate on post-hatch experimental avian necrotic enteritis", VETERINARY PARASITOLOGY, ELSEVIER SCIENCE, AMSTERDAM, NL, vol. 206, no. 3, 27 October 2014 (2014-10-27), pages 115-122, XP029108216, ISSN: 0304-4017, DOI: 10.1016/J.VETPAR.2014.10.025**
- **KYUNGWOO LEE ET AL: "Identification and cloning of two immunogenic proteins, elongation factor Tu (EF-Tu) and pyruvate:ferredoxin oxidoreductase (PFO) of", RESEARCH IN VETERINARY SCIENCE, BRITISH VETERINARY ASSOCIATION, LONDON, GB, vol. 91, no. 3, 20 January 2011 (2011-01-20), pages e80-e86, XP028102751, ISSN: 0034-5288, DOI: 10.1016/J.RVSC.2011.01.017 [retrieved on 2011-01-31]**
- **JI YOUNG PARK ET AL: "Characterization of Clostridium perfringens isolates obtained from 2010 to 2012 from chickens with necrotic enteritis in Korea", POULTRY SCIENCE, vol. 94, no. 6, 3 April 2015 (2015-04-03), pages 1158-1164, XP055415904, Oxford ISSN: 0032-5791, DOI: 10.3382/ps/pev037**

Remarks:
The complete document including Reference Table(s) and the Sequence Listing(s) can be downloaded from the EPO website

**Description**

Field of the Invention

The present invention relates to an *in vitro* method for predicting a necrotic enteritis outbreak in an avian population. More specifically, the present invention provides a method for monitoring the ratio of the amounts of two toxin-encoded genes (*netB / cpa* and *cpa/netB,* resp.) in fecal sample material over time which enables predicting a near-term necrotic entities outbreak.

Background of the Invention

*Clostridium perfringens* is an ubiquitous pathogen that uses an arsenal of toxins to cause histotoxic and intestinal infections in animals and also in humans. *C. perfringens* is a Gram-positive, rod-shaped, spore forming, oxygen-tolerant anaerobe. Not all *C. perfringens* strains are virulent. The virulent *C. perfringens* strains are traditionally classified into five toxin types (A, B, C, D and E), based on the production of four suspected major toxins (alpha, beta, epsilon and iota). Depending on the toxins produced (major and additional toxins like NetB, Cpb2 and others), the *C. perfringens* sub-species specific syndromes/diseases can be induced in different host organisms [Rood, J. I. (1998) "Virulence genes of Clostridium perfringens"; Annual Review of Microbiology 52: 333-360]. The toxins are encoded by polynucleotide sequences located on the chromosome and/or on toxin plasmids [Popoff, M. R. and P. Bouvet (2013). "Genetic characteristics of toxigenic Clostridia and toxin gene evolution" Toxicon 75: 63-89].

As an animal pathogen, *C. perfringens* is responsible for several serious diseases including avian necrotic enteritis, which drains approximately US$ 6 billion/year from the global agricultural system [Wade, B., Keyburn, A.L. (2015), "The true cost of necrotic enteritis" World Poultry 31, 16-17].

Necrotic enteritis (NE) is an enteric disease of poultry that was first described in 1961. NE in chickens manifests as an acute or chronic enterotoxaemia. The acute disease results in significant levels of mortality due to the development of necrotic lesions in the gut wall, whereas the chronic disease leads to a significant loss of productivity and welfare. Early studies on NE suggested that the main virulence factor involved in the disease was the alpha-toxin (known as Cpa or Pic), which has phospholipase C and sphingomyelinase activity [Keyburn, A. L. et al. (2006) "Alpha-toxin of Clostridium perfringens is not an essential virulence factor in necrotic enteritis in chickens", Infection and Immunity 74(11): 6496-6500]. All *C. perfringens* strains harbor the gene encoding the alpha toxin [Rood, J. I. (1998) "Virulence genes of Clostridium perfringens", Annual Review of Microbiology 52: 333-360; Titball, R. W., et al. (1999) "The Clostridium perfringens α-toxin." Anaerobe 5(2): 51-64]. Recent studies however showed that alpha-toxin seems not to be an essential virulence factor since alpha toxin mutant strains were capable of causing NE, which questions the role of alpha-toxin in the disease in general. In more recent studies, the novel pore forming toxin, NetB, has been suggested to play a major key role in the development of this disease [Keyburn, A. L. et al. (2008) "NetS, a new toxin that is associated with avian necrotic enteritis caused by Clostridium perfringens" PLoS Pathogens 4(2)].

NE is known to affect broilers, laying hens, turkeys, and quail. The clinical form is most commonly seen in two to five week- old broilers. Typically, this is also near the time that diets are switched from starter feed to grower feed and near the transition period from the maternal immune system to the adaptive immune system, respectively, so opportunistic *C. perfringens* may take advantage of this transitional period in the intestinal environment and proliferate [Timbermont, L. et al. (2011) "Necrotic enteritis in broilers: An updated review on the pathogenesis." Avian Pathology 40(4):341-347]. WO2016/142146 relates to methods of detecting avian necrotic enteritis.

Since not all *Clostridium perfringens* strains are capable of causing NE, differential analyses are required to differentiate further the NE causing strains. Molecular methods such as PCR, AFLP (amplified fragment length polymorphism) and/or PFGE (pulsed-field gel electrophoresis) are available for the identification of the *Clostridium perfringens* strains. These methods, however, are still limited in their quantitative power of discriminating NE causing strains of *Clostridium perfringens.*

Moreover, a method for predicting an NE outbreak in an avian population is of particular interest as such prediction would enable an early intervention and would offer an advantage of several days to the farmers against the conventional methods for the detection of NE in an avian population. It was thus an urgent need to provide a fast and reliable, non-invasive *ante mortem* method for early detection of a necrotic enteritis outbreak in an avian population.

Summary of the Invention

Accordingly, one object of the present invention is to provide an *in vitro* method for predicting a necrotic enteritis outbreak in an avian population, the method comprising:

a) collecting fecal sample material deriving from the avian population at consecutive points in time; and

b) determining the ratio of the amounts of the marker genes *netB* to *cpa,* contained in the sample material obtained in step a);

wherein a reversion of the ratio of the amounts of *netB* to *cpa* over time ("transition") predicts a near-term necrotic enteritis outbreak.

An additional object of the present invention is the provision of an *in vitro* method for controlling the necrotic enteritis status in an avian population, the method comprising monitoring the ratio of the amounts of the marker genes *netB* to *cpa* contained in fecal samples collected at consecutive points in time, wherein

a) a reversion of the ratio of the amounts of *netB* to cpa over time ("transition") indicates the necessity of a nutritional or therapeutic intervention, and/or
b) a re-reversion of the ratio of the amounts of *netB* to *cpa* over time ("reverse transition") after administering nutritional or therapeutic agents indicates the effectivity of the nutritional or therapeutic intervention.

Disclosed, but not part of the claimed invention, is a multiplex qPCR kit suitable for applying same in the above method, the kit comprising a specific pair of primers for *netB* and a specific pair of primers for *cpa.*

In the following, the crucial aspects of the present invention are described in detail.

Detailed Description of the invention

The inventors have unexpectedly found that a reversion of the ratio of the amounts of the marker genes *netB* to *cpa* (i.e. the ratio of *netB*/*cpa* and *cpa*/*netB,* respectively) occurs consistently prior to the pathological diagnosis of necrotic enteritis in an avian population. That is, said reversion of the ratio of the amounts of the marker genes *netB* to *cpa* can be used as a diagnostic marker to predict the onset and / or the outbreak of necrotic enteritis in an avian flock.

Accordingly, the present invention is directed to an *in vitro* method for predicting a necrotic enteritis outbreak in an avian population, the method comprising:

a) collecting fecal sample material deriving from the avian population at consecutive points in time; and
b) determining the ratio of the amounts of the marker genes *netB* to *cpa,* contained in the sample material obtained in step a);

wherein a reversion of the ratio of the amounts of *netB* to *cpa* over time ("transition") predicts a near-term necrotic enteritis outbreak.

In the context of the present invention, the term "marker gene" includes functional fragments of the respective marker gene; i.e. functional fragments of *netB* and *cpa.*

The term "necrotic enteritis"/NE refers to both, clinical and sub-clinical/latent conditions.

Accordingly, the term "outbreak" is to be understood as sudden or spontaneous occurrence of the disease (necrotic enteritis) in a normal avian population, as well as its induction by the administration of *Clostridium perfringens* alone or in combination with predisposing conditions [Shojadoost, B., Vince, A.R., Prescott, J.F., 2012. The successful experimental induction of necrotic enteritis in chickens by Clostridium perfringens: a critical review. Vet. Res. 43, 74; Fernandes da Costa, S.P., Mot, D., Bokori-Brown, M., Savva, C.G., Basak, A.K., Van Immerseel, F., Titball, R.W., 2013. Protection against avian necrotic enteritis after immunisation with NetB genetic or formaldehyde toxoids. Vaccine 31, 4003-4008; Williams, R.B., Marshall, R.N., La Ragione, R.M., Catchpole, J., 2003. A new method for the experimental production of necrotic enteritis and its use for studies on the relationships between necrotic enteritis, coccidiosis and anticoccidial vaccination of chickens. Parasitol. Res. 90, 19-26; Wu, S.B., Rodgers, N., Choct, M., 2010. Optimized necrotic enteritis model producing clinical and subclinical infection of Clostridium perfringens in broiler chickens. Avian Dis. 54, 1058-1065; Wu S-B, Stanley D, Rodgers N, Swick RA, Moore RJ. Two necrotic enteritis predisposing factors, dietary fishmeal and eimeria infection, induce large changes in the caecal microbiota of broiler chickens. Vet Microbiol 2014;169:188e97].

The inventors have found that the time interval between the reversion of the ratio of the amounts of the marker genes *netB* to *cpa* and the pathological diagnosis of necrotic enteritis using conventional techniques is between one day and five days.

As an example for such reversion or transition, the *netB*/*cpa* ratio may be <1 (corresponding to a *cpa*/*netB* ratio >1) at one day; and at the following day, the *netB*/*cpa* ratio may be >1 (corresponding to a *cpa*/*netB* ratio <1).

In accordance with these findings, the aforementioned method may further comprise
c) identifying the time point where the ratio of the amounts of the marker genes *netB* to *cpa* are reversed ("transition point").

Said transition point may e.g. be determined via graphical analysis. Therefore, two graphs are to be drawn up:

The first graph represents the amount of *netB* vs. the time point of sample collection; and the second graph represents the amount of *cpa* vs. the time point of sample collection. From the point of intersection of these two graphs, the transition point may be read off.

The polynucleotide sequences of *netB* and *cpa* are known in the art. However, for the sake of clarity and completeness, the consensus sequence of *netB* is indicated under SEQ ID NO.: 1 and the consensus sequence of *cpa* is indicated under SEQ ID NO. 2.The *cpa* gene is located on the chromosome of all *C. perfringens* strains (pathogenic and non-pathogenic); whereas the *netB* gene is located on a toxin plasmid of pathogenic (NE inducing) *C. perfringens* strains.

The fecal sample material of step a) may be a composite fecal sample from randomly selected individual samples.

In the context of the present invention, the term "feces" is to be understood as the cloacal defecation product of avian subjects. The fecal sample material is thus gained in non-invasive manner and collected by the sampling techniques described below.

The fecal samples to be taken from a specific avian population are ideally taken at a discrete number of sites within the animal house in order to obtain a pooled sample being representative for the animal population as a whole.

The sample size (i.e. the number of fecal samples to be taken; each sample taken at a specific site within the animal house) has to be determined in view of the actual stocking density, i.e. with the actual number of animals belonging to the avian population to be tested.

The sample size may be calculated using the following formula:

$$ n_0 = \frac{Z^2 pq}{e^2} $$

wherein

$n_0$ is the sample size recommendation

Z is 1.96 for 95% confidence level

p is the estimated portion of the population with the attribute in question q is 1-p, and

e is the confidence interval expressed as decimal.

In general, a minimum of 80 to 100 individual fecal samples are sufficient for most livestock avian populations. As an example, for a broiler flock of 20000 animals, 96 individual samples are required for a confidence level of 95%.

For obtaining the pooled fecal sample material as required in step a), several sampling methods may be used. In one embodiment, the pooled fecal sample is obtained by systematic grid sampling (systematic random sampling). For this method, the animal house or area in which the avian population is kept is divided in a grid pattern of uniform cells or sub-areas based on the desired number of individual fecal samples (i.e. the sample size). Then, a random sample collection site is identified within the first grid cell and a first sample is taken at said site. Finally, further samples are obtained from adjacent cells sequentially - e.g. in a serpentine, angular or zig-zag fashion - using the same relative location within each cell. A random starting point can be obtained with a dice or a random number generator.

The above process may optionally be repeated for replicate samples. That is, a new random position is established for the single collection point to be repeated in all of the cells. By analyzing replicate samples, variabilities in the estimate of the mean provided by the original samples may be determined.

Accordingly, the aforementioned methods may further comprise the following sub-steps:

(a1) dividing the animal house or the area in which the animal population is kept in a grid pattern of an equal number of uniform cells;
(a2) identifying at least one random sample collection site within the first cell and taking one first sample at said random sample collection site; and
(a3) sequentially collecting individual fecal samples in the remaining cells using the same relative sample collection sites within each cell;
and optionally
(a4) repeating steps (a2) and (a3) for at least one replicate sample.

The sample size corresponds to the number of cells in the grid pattern in case one sample is to be taken per cell. In general, in case x samples are to be taken per cell, the sample size is the number of cells, divided by x.

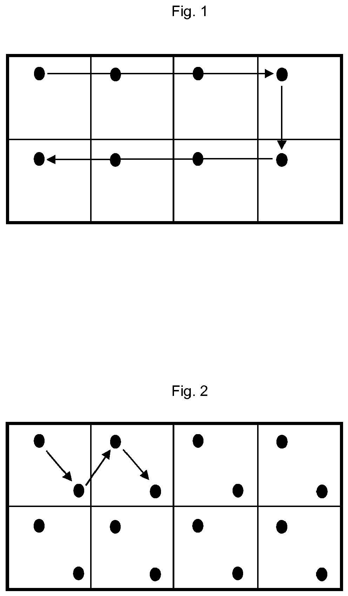

The systematic grid sampling method can be easily implemented in the field. Thereby, over- or underrepresentation of subareas can be avoided. Systematic grid sampling patterns according to the present invention are exemplified in Fig. 1 and Fig. 2.

Another sampling method is stratified random sampling (i.e. random sampling within a grid). Herein, samples are obtained sequentially from adjacent grid cells, but the location of the sample within each cell is random.

Alternatively, the samples may be taken by simple random sampling, where the samples are taken from random locations (without gridding) across the area in which the animals are kept. For this method, a formal approach for determining the random sample locations must be used, e.g. based upon a random number generator.

The samples may be collected manually with a spatula or a similar device and are immediately transferred into a sample collection vessel or tube.

In an alternative embodiment, the pooled fecal sample may be obtained using the overshoe method while walking through the house using a route that will produce representative samples for all parts of the house or the respective sector. Such route may e.g. be uniformly shaped serpentines or sinuous lines, angular lines or zigzag lines. Boot swabs being sufficiently absorptive to soak up moisture are particularly suitable. However, tube gauze socks are also acceptable. Suitable sample masses for the individual samples taken are, for example 0.1 to 20 g, in particular 0.2 to 10 g, preferably 0.5 to 5 g. The samples may be collected manually with a spatula, a litter grab or a similar device.

After finishing sample collection, the sample material has to be homogenized. The skilled artisan is aware of suitable, commonly used homogenization techniques. The thus-obtained pooled sample may be diluted and/or stabilized. Sample stabilization in this context means protecting the nucleic acid material contained in the sample against nucleases in solution, e.g. by using a buffer solution comprising nuclease inhibitors.

The sample material is to be collected at consecutive points in time. The fecal sample material may be collected and analyzed on a weekly, daily, or hourly basis. For example, fecal test samples may be collected and analyzed on a daily basis from birth to slaughter.

The avian population preferably is an avian flock. The avian flock according to the invention is preferably poultry. Preferred poultry according to the invention are chickens, turkeys, ducks and geese. The poultry can be optimized for producing young stock. This type of poultry is also referred to as parent and grandparent animals. Preferred parent and grandparent animals are, accordingly, (grand) parent broilers, (grand) parent ducks, (grand) parent turkeys and (grand) parent geese.

The poultry according to the invention can also be selected from fancy poultry and wild fowl. Preferred fancy poultry or wild fowl are peacocks, pheasants, partridges, guinea fowl, quails, capercailzies, goose, pigeons and swans. Further preferred poultry according to the invention are ostriches and parrots. Most preferred poultry according to the invention are broilers.

For broiler flocks, fecal samples may be collected and analyzed on a daily basis during the initial growth phase (starter phase, day 5 to day 10), and/or during the enhanced growth phase (day 11 to day 18) and, optionally, also on a later stage.

In one embodiment, the fecal sample material, in particular fecal sample material, from the broiler flock is collected and analyzed on a daily basis starting from day 10.

The marker genes *netB* and *cpa* may be isolated from the fecal samples prior to quantification.

Polynucleotide isolation can for example, be performed via extraction using the Cetyltrimethylammoniumbromid (CTAB) method or by diverse commercial nucleic acid extraction kits, in which cell lysis is achieved either through chemical lysis and/or by mechanical cell disruption and nucleic acid is captured on silica matrices or on silica-cladded magnetic beads. Commercial extraction kits specialized on fecal material or harsh material are particularly suitable.

The marker genes may be detected and/or quantified by commonly known methods such as sequencing, hybridization or various PCR techniques known in the art.

In an alternative embodiment, the marker genes contained in the animal sample can be quantified directly, for example via PCR, qPCR, sequencing or hybridization techniques.

In one specific embodiment, the ratio of the amounts of the marker genes *netB* to *cpa,* or of homologues or functional fragments of these marker genes, contained in the sample material obtained in step a) are determined via qPCR.

In the above-specified inventive methods, one or more oligonucleotides selected from the group consisting of

a) oligonucleotides having a sequence identity of at least 80%, preferably at least 85, 90 or 95%, most preferably 100%, to the polynucleotide as depicted in SEQ ID NO.:3;

b) oligonucleotides having a sequence identity of at least 80%, preferably at least 85, 90 or 95%, most preferably 100%, to the polynucleotide as depicted in SEQ ID NO.: 4;

c) oligonucleotides having a sequence identity of at least 80%, preferably at least 85, 90 or 95%, most preferably 100%, to the polynucleotide as depicted in SEQ ID NO.: 5;

d) oligonucleotides having a sequence identity of at least 80%, preferably at least 85, 90 or 95%, most preferably 100%, to the polynucleotide as depicted in SEQ ID NO.: 6;

e) oligonucleotides having a sequence identity of at least 80%, preferably at least 85, 90 or 95%, most preferably 100%, to the polynucleotide as depicted in SEQ ID NO.: 7;

f) oligonucleotides having a sequence identity of at least 80%, preferably at least 85, 90 or 95%, most preferably 100%, to the polynucleotide as depicted in SEQ ID NO.:8;

g) oligonucleotides being complementary to the oligonucleotides according to (a) to (f);

h) oligonucleotides comprising any one of the oligonucleotides according to (a) to (g) and being elongated by not more than 5 base pairs compared to the oligonucleotides according to (a) to (g);

may be used as a PCR primer and/or as a PCR probe.

Therein, the polynucleotide as depicted in SEQ ID NO.: 3 is a PCR primer (fwd) for detecting *netB*. The polynucleotide as depicted in SEQ ID NO.: 4 is a PCR primer (rev) for detecting *netB*. The polynucleotide as depicted in SEQ ID NO.: 5 is a PCR probe for detecting *netB*.

Further, the polynucleotide as depicted in SEQ ID NO.: 6 is a PCR primer (fwd) for detecting *cpa*.

The polynucleotide as depicted in SEQ ID NO.: 7 is a PCR primer (rev) for detecting *cpa*. The polynucleotide as depicted in SEQ ID NO.: 8 is a PCR probe for detecting *cpa*.

In accordance with the above, the present disclosure is further directed to the use of oligonucleotides selected from the group consisting of

a) oligonucleotides having a sequence identity of at least 80%, preferably at least 85, 90 or 95%, most preferably 100%, to the polynucleotide as depicted in SEQ ID NO.:3;

b) oligonucleotides having a sequence identity of at least 80%, preferably at least 85, 90 or 95%, most preferably 100%, to the polynucleotide as depicted in SEQ ID NO.: 4;

c) oligonucleotides having a sequence identity of at least 80%, preferably at least 85, 90 or 95%, most preferably 100%, to the polynucleotide as depicted in SEQ ID NO.: 5;

d) oligonucleotides having a sequence identity of at least 80%, preferably at least 85, 90 or 95%, most preferably 100%, to the polynucleotide as depicted in SEQ ID NO.: 6;

e) oligonucleotides having a sequence identity of at least 80%, preferably at least 85, 90 or 95%, most preferably 100%, to the polynucleotide as depicted in SEQ ID NO.: 7;

f) oligonucleotides having a sequence identity of at least 80%, preferably at least 85, 90 or 95%, most preferably 100%, to the polynucleotide as depicted in SEQ ID NO.:8;

g) oligonucleotides being complementary to the oligonucleotides according to (a) to (f);

h) oligonucleotides comprising any one of the oligonucleotides according to (a) to (g) and being elongated by not more than 5 base pairs compared to the oligonucleotides according to (a) to (g);

for early detection of a necrotic enteritis outbreak in an avian population.

The present invention provides the above-described non-invasive methods for predicting a NE outbreak, which can be performed *ante mortem*. This enables the farmer to take measures against the necrotic enteritis outbreak at an early stage.

Accordingly, the present invention also pertains to the use of any one of the aforementioned methods for determining the necessity of nutritional or therapeutic interventions.

Such interventions or measures include feeding or administering health-promoting substances, such as zootechnical feed additives, or therapeutic agents. The term "administering" or related terms includes oral administration. Oral administration may be via drinking water, oral gavage, aerosol spray or animal feed. The term "zootechnical feed additive" refers to any additive used to affect favorably the performance of animals in good health or used to affect favorably the environment. Examples for zootechnical feed additives are digestibility enhancers, i.e. substances which, when fed to animals, increase the digestibility of the diet, through action on target feed materials; gut flora stabilizers; micro-organisms or other chemically defined substances, which, when fed to animals, have a positive effect on the gut flora; or substances which favorably affect the environment. Preferably, the health-promoting substances are selected from the group consisting of probiotic agents, prebiotic agents, botanicals, organic/fatty acids, bacteriophages and bacteriolytic enzymes or any combinations thereof.

Further, the inventors have found that a re-reversion ("reverse transition") of the ratio of the amounts of *netB* to *cpa* over time indicates regression or disappearance of necrotic enteritis in the avian population. Said regression or disappearance may occur naturally (as a spontaneous recovery) or may be caused by therapeutic or nutritional interventions.

Accordingly, the present invention provides an *in vitro* method for controlling the necrotic enteritis status in an avian population, the method comprising monitoring the ratio of the amounts of the marker genes *netB* to *cpa* contained in fecal samples collected at consecutive points in time, wherein

a) a reversion of the ratio of the amounts of *netB* to *cpa* over time ("transition") indicates the necessity of a nutritional or therapeutic intervention, and

b) a re-reversion of the ratio of the amounts of *netB* to *cpa* over time ("reverse transition") after administering nutritional or therapeutic agents indicates the effectivity of the nutritional or therapeutic intervention.

The time point of the re-reversion ("reverse transition point") may be determined graphically as described in the above for the transition point.

The term "controlling the necrotic enteritis status" is to be understood as determining whether or not there is any indication for a necrotic enteritis outbreak or for a regression/disappearance of necrotic enteritis, respectively.

Suitable sample materials and methods of sample collection for this method are as described in the above.

The nutritional or therapeutic intervention may involve administering substances selected from the group consisting of probiotic agents, prebiotic agents, botanicals, organic/fatty acids, bacteriophages and bacteriolytic enzymes or any combinations thereof. Probiotics are particularly preferred.

Disclosed, but not part of the claimed invention, are probiotic agents for use in the treatment of necrotic enteritis, wherein the necrotic enteritis outbreak is detected by any one of the aforementioned methods.

As an example for the above methods for controlling the necrotic enteritis status in an avian population, necrotic enteritis is diagnosed based on the reversion of the ratio of the amounts of *netB* to *cpa* over time; e.g. the *netB*/*cpa* ratio turns from a value <1 to a value >1. Immediately after diagnosis, the farmer intervenes e.g. by administering probiotic agents. The ratio of the amounts of *netB* to *cpa* contained in fecal samples collected at consecutive points in time are further monitored. In case the intervention is effective, the ratio of *netB* to *cpa* reverses again, e.g. the *netB*/*cpa* ratio turns from a value >1 to a value <1.

In one embodiment of the above methods for controlling the necrotic enteritis status in an avian population, one or more oligonucleotides selected from the group consisting of

a) oligonucleotides having a sequence identity of at least 80%, preferably at least 85, 90 or 95%, most preferably 100%, to the polynucleotide as depicted in SEQ ID NO:3;

b) oligonucleotides having a sequence identity of at least 80%, preferably at least 85, 90 or 95%, most preferably 100%, to the polynucleotide as depicted in SEQ ID NO: 4;

c) oligonucleotides having a sequence identity of at least 80%, preferably at least 85, 90 or 95%, most preferably 100%, to the polynucleotide as depicted in SEQ ID NO: 5;

d) oligonucleotides having a sequence identity of at least 80%, preferably at least 85, 90 or 95%, most preferably 100%, to the polynucleotide as depicted in SEQ ID NO: 6;

e) oligonucleotides having a sequence identity of at least 80%, preferably at least 85, 90 or 95%, most preferably 100%, to the polynucleotide as depicted in SEQ ID NO: 7;

f) oligonucleotides having a sequence identity of at least 80%, preferably at least 85, 90 or 95%, most preferably 100%, to the polynucleotide as depicted in SEQ ID NO:8;

g) oligonucleotides being complementary to the oligonucleotides according to (a) to (f);

h) oligonucleotides comprising any one of the oligonucleotides according to (a) to (g) and being elongated by not more than 5 base pairs compared to the oligonucleotides according to (a) to (g);

are used as a PCR primer and/or as a PCR probe.

In accordance with the above, the present disclosure further pertains to the use of oligonucleotides selected from the group consisting of

a) oligonucleotides having a sequence identity of at least 80%, preferably at least 85, 90 or 95%, most preferably 100%, to the polynucleotide as depicted in SEQ ID NO.:3;

b) oligonucleotides having a sequence identity of at least 80%, preferably at least 85, 90 or 95%, most preferably 100%, to the polynucleotide as depicted in SEQ ID NO.: 4;

c) oligonucleotides having a sequence identity of at least 80%, preferably at least 85, 90 or 95%, most preferably 100%, to the polynucleotide as depicted in SEQ ID NO.: 5;

d) oligonucleotides having a sequence identity of at least 80%, preferably at least 85, 90 or 95%, most preferably 100%, to the polynucleotide as depicted in SEQ ID NO.: 6;

e) oligonucleotides having a sequence identity of at least 80%, preferably at least 85, 90 or 95%, most preferably 100%, to the polynucleotide as depicted in SEQ ID NO.: 7;

f) oligonucleotides having a sequence identity of at least 80%, preferably at least 85, 90 or 95%, most preferably 100%, to the polynucleotide as depicted in SEQ ID NO.:8;

g) oligonucleotides being complementary to the oligonucleotides according to (a) to (f);

h) oligonucleotides comprising any one of the oligonucleotides according to (a) to (g) and being elongated by not more than 5 base pairs compared to the oligonucleotides according to (a) to (g);

for determining the effectivity of nutritional or therapeutic interventions.

Disclosed, but not part of the claimed invention, is a diagnostic multiplex qPCR kit for determining the ratio of the amounts of *netB* to *cpa* and for monitoring the ratio of the amounts of *netB* to *cpa* over time, respectively.

Said kit comprises a primer pair for detecting *netB* and a primer pair for detecting *cpa*,

> wherein the primer pair for *netB* comprises
> oligonucleotides having a sequence identity of at least 80%, preferably at least 85, 90 or 95%, most preferably 100%, to the polynucleotide as depicted in SEQ ID NO.:3, and
> oligonucleotides having a sequence identity of at least 80%, preferably at least 85, 90 or 95%, most preferably 100%, to the polynucleotide as depicted in SEQ ID NO.: 4;
> and the primer pair for *cpa* comprises
> oligonucleotides having a sequence identity of at least 80%, preferably at least 85, 90 or 95%, most preferably 100%, to the polynucleotide as depicted in SEQ ID NO.: 6, and
> oligonucleotides having a sequence identity of at least 80%, preferably at least 85, 90 or 95%, most preferably 100%, to the polynucleotide as depicted in SEQ ID NO.: 7.

Optionally, the multiplex qPCR kit according to the present disclosure may additionally comprise one or more probes for detecting *netB* and/or one or more probes for detecting *cpa*.

In one embodiment, the multiplex qPCR kit comprises - in addition to the abovementioned primer pairs for *netB* and *cpa* - a probe for detecting *netB* and a probe for detecting *cpa*,

> wherein the probe for detecting *netB* comprises
> oligonucleotides having a sequence identity of at least 80%, preferably at least 85, 90 or 95%, most preferably 100%, to the polynucleotide as depicted in SEQ ID NO.: 5;
> and the probe for detecting *cpa* comprises
> oligonucleotides having a sequence identity of at least 80%, preferably at least 85, 90 or 95%, most preferably 100%, to the polynucleotide as depicted in SEQ ID NO.:8.

The kit may further comprise buffer solutions, such as PCR buffer; magnesia salts; deoxy nucleotide triphosphates (dNTPs). The kit may also include elements such as sample collection tubes, reagents to isolate the nucleic acids and/or instructions for its use.

Applications of the methods according to the disclosure are for example ((i) aiding in the diagnosis and/or prognosis of avian necrotic enteritis, (ii) monitoring the progress or reoccurrence of avian necrotic enteritis (iii) aiding in the evaluation of treatment efficacy for an animal population undergoing or contemplating treatment, or (iv) controlling (therapeutic) vaccination efficiency against *C. perfringens* induced avian necrotic enteritis.

Applications of the methods according to the present disclosure in particular help to avoid loss in animal performance like weight gain and feed conversion.

In the following, the invention is illustrated by non-limiting examples and exemplifying embodiments.

Examples

About 20,000 broiler were randomly assigned to broiler houses as part of the normal chicken placement procedures of the company, in accordance to the American Humane Association certified program, which limits density to 6.2 pounds /square foot at slaughter, including substantial management, and auditing needs. All flocks were managed according to company's standard protocols, which are in line with breeder's recommendations for lighting, temperature, and ventilation. Feeds consisted of basal diet (corn and soy) adjusted for birds requirements for starter, grower & finisher feeds. General flock conditions were monitored daily: the availability of feed and water, temperature control, and any unusual conditions. Dead bird were removed and necropsied to determine cause of death and debilitated birds were culled to avoid further suffering.

Sample collection

Fecal samples and flock performance data from several standard broiler live production processes were collected daily from days 10/11 to 24/25 for a period of 2 years. During this period, three flocks (Examples 1, 2, & 3) were diagnosed as necrotic enteritis positive (outbreak flocks) flocks by mortality spike during NE disease window and the observation of NE typical lesions in the guts of necropsied dead birds by the veterinarian. All fecal samples collected from these NE outbreak flocks were processed separately, according to the instructions of Evonik's proprietary sample processing and qPCR workflow.

At each collection time point or event, 24 individual samples were picked up from each quadrant of the house with a plastic tong, walking each quadrant in a zig-zag fashion. To avoid cross contamination of samples, new sterile tong was used for each house as well as prescribed biosecurity measures were observed. Furthermore, debris such as wood shavings, litter, etc., were removed from the samples before all samples from the 4 quadrants were composited to form a single pooled sample (consisting of 96 individual fecal samples) in a sterile sample collection bag. The samples were placed an ice and transferred to the laboratory for storage at -80° C.

DNA extraction

Each bag with the pooled 96 samples was allow to thaw slowly at room temperature; then, the feces were transferred into a sterile container and mixed thoroughly with a sterile tongue depressor. Five (5) grams of the homogenized sample were transferred to a proprietary sample collection tube, containing 20 ml of stabilization buffer and glass beads. Fecal samples in the sample collection tubes are stable for up to 7 days at +15°C to + 30°C.

The tube containing the fecal sample was incubated at 70°C for 20 minutes in a water bath. The tube was then transferred to a Poly Mix Mill (bead beater) for homogenization at 20 Hz for 15 minutes. At the end of the homogenization, the sample was centrifuged at 2000g for 5 minutes, and 500 $\mu$l of the supernatant was used for DNA extraction. DNA extraction was performed with the King Fisher Flex system (Thermo Fisher, USA), adhering to the protocol of Evonik's proprietary fecal extraction kit.

The King Fisher instrument was prepared by uploading a predefined program ("Cper_Extraction_01") defining the various steps of the extraction process; sampling tips, DNA elution plate, wash plates and sample plate were prepared as described below.

A 96 tips comb was inserted in an empty deep well plate and placed it in the instrument. This was followed by the introduction of 100$\mu$l of the elute buffer in an elution plate and this plate was also placed in the instrument. Furthermore, 500 $\mu$l of wash buffers 3, 2 and 1 where place in each well of 3 different wash plates respectively, and these plates were placed on the instrument in the same order. Finally, 300 $\mu$l of lysis buffer, 25 $\mu$l magnetic beads, 20 $\mu$l Enhancer, 10 $\mu$l internal control and 500 $\mu$l of the supernatant from the fecal sample were added to each well of a sample plate. After placing the sample plate on the instrument, the extraction was started by pressing the start button.

DNA Quantification

For the quantification of markers in the DNA, a 20 $\mu$l master mix consisting of 5 $\mu$l Master A, 15 $\mu$l master B and 1 $\mu$l of IC (internal control) was prepared according to the instruction of proprietary Real-Time PCR detection kit of Evonik Nutrition & Care GmbH per reaction. Enough master mix was prepared to accommodate the running of all samples, non-template controls (NTC) and 4 standards (S1 to S4) in duplicates. 20 $\mu$l of the master mix were dispensed into individual wells of a 96 well plate. Then, a 10 $\mu$l of the extracted DNA sample was transferred into each well. 10 $\mu$l of the respective standard and 1 $\mu$l of IC were transferred to each standard well accordingly. To prepare a NTC, 10 $\mu$l of sterile nuclease free water and 1 $\mu$l of IC were transferred to the NTC wells each. The contents of the plate were mixed thoroughly with a multi-channel pipet, and the plate was sealed with a Clear Weld Seal Mark II foil. film. The plate was centrifuged for 30 seconds at 1000 g (-3000 rpm). Finally, the plate was run on a CFX96 real time PCR instrument (Bio Rad, Germany) with the following PCR conditions: 45 cycles of denaturation at 95°C for 15 seconds, annealing at 58°C for 45 seconds and extension at 72°C for 15 seconds. Data were acquired during the amplification phase of the QPCR run. At the end of the run, data received from the BioRad CFX96 were preprocessed with the Bio- Rad CFX Manager 3.1 and exported to Excel 2013 for further analysis. The quantification of markers in samples were determined from the standard curve constructed with standard solutions (S1 to S4) containing equal concentrations of both targets. The concentrations of *netB* and *cpa* in S1, S2, S3 and S4 are $10^4$ copies/$\mu$l, $10^3$ copies/$\mu$l, $10^2$ copies/$\mu$l and $10^1$ copies/$\mu$l respectively. The log of the standards were plotted along the x-axis, while the Ct (cycle thresholds) were plotted along the y-axis. The resulting linear regression line [y=mx + b or Ct= m (log quantity) + b] was used to determine the concentrations of the targets in the sample tested.

List of primers and probe used for the qPCR to quantify levels of expression of targets:

| Target | Primers and Probes (where applicable) | Probe reporter |
|--------|----------------------------------------|----------------|
| *netB* | Forward: 5'- TATACTTCTAGTGATACCGC -3' (SEQ ID NO.: 3) | |
| | Reverse: 5'- ATCAGAATGAGGATCTTCAA -3' (SEQ ID NO.: 4) | |
| | Probe: 5' TCACATAAAGGTTGGAAGGCAAC-3' (SEQ ID NO.: 5) | FAM |
| *cpa* | Forward: 5'- TACATATCAACTAGTGGTGA -3' (SEQ ID NO.: 6) | |

(continued)

| Target | Primers and Probes (where applicable) | Probe reporter |
|---|---|---|
| | Reverse: 5'- ATTCTTGAGTTTTTCCATCC -3' (SEQ ID NO.: 7) | |
| | Probe: 5'- TGGAACAGATGACTACATGTATTTTGG-3 (SEQ ID NO.: 8) | Cy5 |

Example 1:

| Day | Marker | Cq | mean Cq | Starting quantity for 1g feces | Log10 | Mean | netB/cpa (for log 10 values) |
|---|---|---|---|---|---|---|---|
| | netB | 28,05 | 27,955 | 4,49E+04 | 4,65E+00 | 4,68E+00 | 0,99 |
| | | 27,86 | | 5,14E+04 | 4,71E+00 | | |
| | cpa | 28,86 | 28,795 | 5,04E+04 | 4,70E+00 | 4,72E+00 | |
| **13** | | 28,73 | | 5,50E+04 | 4,74E+00 | | |
| | netB | 28,41 | 28,365 | 3,48E+04 | 4,54E+00 | 4,55E+00 | 0,83 |
| | | 28,32 | | 3,70E+04 | 4,57E+00 | | |
| | cpa | 26,32 | 26,26 | 2,94E+05 | 5,47E+00 | 5,49E+00 | |
| **14** | | 26,2 | | 3,19E+05 | 5,50E+00 | | |
| | netB | 25,19 | 25,195 | 3,42E+05 | 5,53E+00 | 5,53E+00 | 1,04 |
| | | 25,2 | | 3,40E+05 | 5,53E+00 | | |
| | cpa | 26,88 | 26,875 | 1,99E+05 | 5,30E+00 | 5,30E+00 | |
| **15** | | 26,87 | | 2,00E+05 | 5,30E+00 | | |
| | netB | 29,02 | 29,035 | 2,26E+04 | 4,35E+00 | 4,35E+00 | 1,04 |
| | | 29,05 | | 2,20E+04 | 4,34E+00 | | |
| | cpa | 30,65 | 30,64 | 14570 | 4,16E+00 | 4,17E+00 | |
| **16** | | 30,63 | | 14690 | 4,17E+00 | | |
| | netB | 29,55 | 29,395 | 1,54E+04 | 4,19E+00 | 4,24E+00 | 1,08 |
| | | 29,24 | | 1.93E+04 | 4,28E+00 | | |
| | cpa | 31,64 | 31,42 | 7,32E+03 | 3,86E+00 | 3,93E+00 | |
| **17** | | 31,2 | | 9,92E+03 | 4,00E+00 | | |
| | netB | 28,79 | 28,75 | 2,66E+04 | 4,42E+00 | 4,44E+00 | 1,06 |
| | | 28,71 | | 2,82E+04 | 4,45E+00 | | |
| | cpa | 30,59 | 30,585 | 1,52E+04 | 4,18E+00 | 4,18E+00 | |
| **20** | | 30,58 | | 1.53E+04 | 4,18E+00 | | |
| | netB | 24,46 | 24,28 | 5,75E+05 | 5,76E+00 | 5,81E+00 | 1,05 |
| | | 24,1 | | 7,41E+05 | 5,87E+00 | | |
| | cpa | 26,21 | 26,06 | 3,16E+05 | 5,50E+00 | 5,55E+00 | |
| **21** | | 25,91 | | 3,89E+05 | 5,59E+00 | | |
| | netB | 28,37 | 28,285 | 3,58E+04 | 4,55E+00 | 4,58E+00 | 1,05 |
| | | 28,2 | | 4,03E+04 | 4,60E+00 | | |
| | cpa | 30,09 | 29,985 | 2,14E+04 | 4,33E+00 | 4,36E+00 | |
| **22** | | 29,88 | | 2,49E+04 | 4,40E+00 | | |

(continued)

| Day | Marker | Cq | mean Cq | Starting quantity for 1g feces | Log10 | Mean | netB/cpa (for log 10 values) |
|---|---|---|---|---|---|---|---|
| | netB | 25,96 | 26,09 | 1,98E+05 | 5,30E+00 | 5,26E+00 | 1,08 |
| | | 26,22 | | 1.65E+05 | 5,22E+00 | | |
| | cpa | 28,12 | 28,26 | 8,38E+04 | 4,92E+00 | 4,88E+00 | |
| **23** | | 28,4 | | 6,93E+04 | 4,84E+00 | | |
| | netB | 24,39 | 24,405 | 6,05E+05 | 5,78E+00 | 5,78E+00 | 1,05 |
| | | 24,42 | | 5,93E+05 | 5,77E+00 | | |
| | cpa | 26,08 | 26,155 | 3,46E+05 | 5,54E+00 | 5,52E+00 | |
| **24** | | 26,23 | | 3,12E+05 | 5,49E+00 | | |

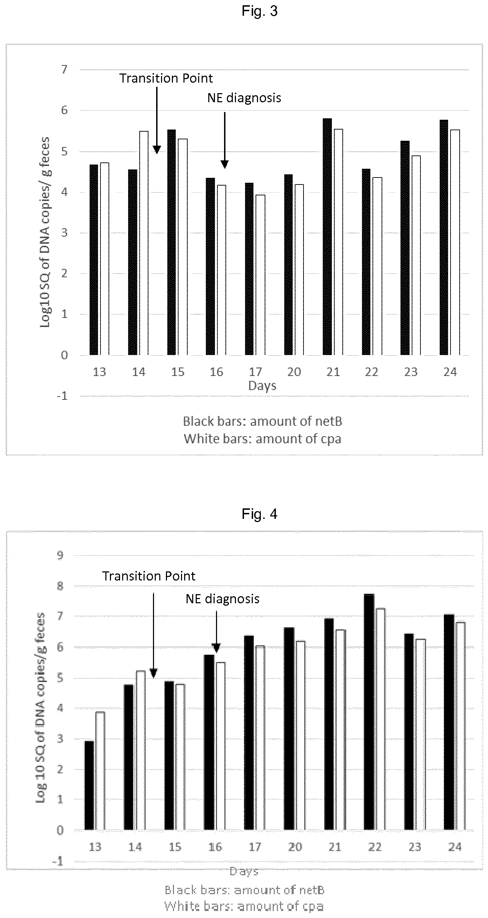

In this Example, the reversion of the ratio of the amounts of *netB* to *cpa* (Transition Point) occurred between day 14 and day 15. The outbreak of necrotic enteritis was established by veterinarian diagnosis (necropsy) on day 16.
Graphical presentation of the data of Example 1 may be found in Fig. 3.

Example 2:

| Day | Marker | Cq | mean Cq | Starting quantity for 1g feces | Log10 | Mean | netB/cpa (for log 10 values) |
|---|---|---|---|---|---|---|---|
| | netB | 33,55 | 33,73 | 8,98E+02 | 2,95E+00 | 2,90E+00 | 0,75 |
| | | 33,91 | | 6,97E+02 | 2,84E+00 | | |
| | cpa | 31,94 | 31,62 | 5,92E+03 | 3,77232171 | 3,86977098 | |
| **13** | | 31,3 | | 9,27E+03 | 3,96722026 | | |
| | netB | 27,71 | 27,705 | 5,73E+04 | 4,76E+00 | 4,76E+00 | 0,92 |
| | | 27,7 | | 5,77E+04 | 4,76E+00 | | |
| | cpa | 27,24 | 27,225 | 1,55E+05 | 5,19E+00 | 5,19E+00 | |
| **14** | | 27,21 | | 1.58E+05 | 5,20E+00 | | |
| | netB | 27,28 | 27,33 | 7,76E+04 | 4,89E+00 | 4,87E+00 | 1,02 |
| | | 27,38 | | 7,22E+04 | 4,86E+00 | | |
| | cpa | 28,6 | 28,57 | 6,05E+04 | 4,78E+00 | 4,79E+00 | |
| **15** | | 28,54 | | 6,26E+04 | 4,80E+00 | | |
| | netB | 24,54 | 24,505 | 5,45E+05 | 5,74E+00 | 5,75E+00 | 1,05 |
| | | 24,47 | | 5,72E+05 | 5,76E+00 | | |
| | cpa | 26,33 | 26,26 | 290400 | 5,46E+00 | 5,49E+00 | |
| **16** | | 26,19 | | 321900 | 5,51E+00 | | |
| | netB | 22,49 | 22,5 | 2,34E+06 | 6,37E+00 | 6,37E+00 | 1,06 |
| | | 22,51 | | 2,30E+06 | 6,36E+00 | | |
| | cpa | 24,55 | 24,485 | 1.00E+06 | 6,00E+00 | 6,02E+00 | |
| **17** | | 24,42 | | 1.10E+06 | 6,04E+00 | | |
| | netB | 21,74 | 21,6 | 3,99E+06 | 6,60E+00 | 6,64E+00 | 1,07 |
| | | 21,46 | | 4,87E+06 | 6,69E+00 | | |
| | cpa | 24,16 | 23,95 | 1,32E+06 | 6,12E+00 | 6,18E+00 | |
| **20** | | 23,74 | | 1.75E+06 | 6,24E+00 | | |

(continued)

| Day | Marker | Cq | mean Cq | Starting quantity for 1g feces | Log10 | Mean | netB/cpa (for log 10 values) |
|---|---|---|---|---|---|---|---|
| | netB | 20,76 | 20,715 | 8,00E+06 | 6,90E+00 | 6,92E+00 | 1,05 |
| | | 20,67 | | 8,50E+06 | 6,93E+00 | | |
| | cpa | 22,7 | 22,665 | 3,61E+06 | 6,56E+00 | 6,57E+00 | |
| 21 | | 22,63 | | 3,81E+06 | 6,58E+00 | | |
| | netB | 18,1 | 18,12 | 5,31E+07 | 7,73E+00 | 7,72E+00 | 1,06 |
| | | 18,14 | | 5,15E+07 | 7,71E+00 | | |
| | cpa | 20,41 | 20,405 | 1,78E+07 | 7,25E+00 | 7,25E+00 | |
| 22 | | 20,4 | | 1.79E+07 | 7,25E+00 | | |
| | netB | 22,4 | 22,295 | 2,50E+06 | 6,40E+00 | 6,43E+00 | 1,03 |
| | | 22,19 | | 2,89E+06 | 6,46E+00 | | |
| | cpa | 23,8 | 23,725 | 1,68E+06 | 6,23E+00 | 6,25E+00 | |
| 23 | | 23,65 | | 1,87E+06 | 6,27E+00 | | |
| | netB | 20,3 | 20,27 | 1,11E+07 | 7,05E+00 | 7,05E+00 | 1,03 |
| | | 20,24 | | 1,16E+07 | 7,06E+00 | | |
| | cpa | 21,8 | 21,825 | 6,77E+06 | 6,83E+00 | 6,82E+00 | |
| 24 | | 21,85 | | 6,54E+06 | 6,82E+00 | | |

In this Example, the reversion of the ratio of the amounts of *netB* to *cpa* (Transition Point) occurred between day 14 and day 15. The outbreak of necrotic enteritis was established by veterinarian diagnosis (necropsy) on day 16.
Graphical presentation of the data of Example 1 may be found in Fig. 4.

Example 3:

| Day | Marker | Cq | mean Cq | Starting quantity for 1g feces | Log10 | Mean | netB/cpa (for log 10 values) |
|---|---|---|---|---|---|---|---|
| | netB | 30,08 | 30,15 | 1,06E+04 | 4,03E+00 | 4,00E+00 | 0,81 |
| | | 30,22 | | 9,58E+03 | 3,98E+00 | | |
| | cpa | 28,07 | 28,01 | 8,72E+04 | 4,94E+00 | 4,96E+00 | |
| 11 | | 27,95 | | 9,47E+04 | 4,98E+00 | | |
| | netB | 31,33 | 31,295 | 4,37E+03 | 3,63998425 | 3,65061443 | 0,70 |
| | | 31,26 | | 4,58E+03 | 3,66124461 | | |
| | cpa | 27,29 | 27,255 | 1,50E+05 | 5,17E+00 | 5,19E+00 | |
| 12 | | 27,22 | | 1.57E+05 | 5,20E+00 | | |
| | netB | 29,21 | 29,25 | 1,96E+04 | 4,29E+00 | 4,28E+00 | 0,81 |
| | | 29,29 | | 1.85E+04 | 4,27E+00 | | |
| | cpa | 26,86 | 26,91 | 2,02E+05 | 5,31E+00 | 5,29E+00 | |
| 13 | | 26,96 | | 1.88E+05 | 5,27E+00 | | |
| | netB | 30,6 | 30,53 | 7,33E+03 | 3,87E+00 | 3,89E+00 | 0,97 |
| | | 30,46 | | 8,09E+03 | 3,91E+00 | | |
| | cpa | 31,15 | 31,15 | 10230 | 4,01E+00 | 4,01E+00 | |
| 14 | | 31,15 | | 10240 | 4,01E+00 | | |
| | netB | 22,17 | 22,13 | 2,94E+06 | 6,47E+00 | 6,48E+00 | 1,05 |

(continued)

| Day | Marker | Cq | mean Cq | Starting quantity for 1g feces | Log10 | Mean | netB/cpa (for log 10 values) |
|---|---|---|---|---|---|---|---|
| | | 22,09 | | 3,11E+06 | 6,49E+00 | | |
| | cpa | 24,13 | 24 | 1,34E+06 | 6,13E+00 | 6,17E+00 | |
| 17 | | 23,87 | | 1.60E+06 | 6,20E+00 | | |
| | netB | 27,52 | 27,58 | 6,56E+04 | 4,82E+00 | 4,80E+00 | 1,06 |
| | | 27,64 | | 5,99E+04 | 4,78E+00 | | |
| | cpa | 29,33 | 29,445 | 3,62E+04 | 4,56E+00 | 4,52E+00 | |
| 18 | | 29,56 | | 3,09E+04 | 4,49E+00 | | |
| | netB | 25,08 | 24,865 | 3,71E+05 | 5,57E+00 | 5,64E+00 | 1,09 |
| | | 24,65 | | 5,02E+05 | 5,70E+00 | | |
| | cpa | 27,54 | 27,25 | 1,26E+05 | 5,10E+00 | 5,19E+00 | |
| 19 | | 26,96 | | 1.88E+05 | 5,27E+00 | | |
| | netB | 22,27 | 21,87 | 2,73E+06 | 6,44E+00 | 6,56E+00 | 1,07 |
| | | 21,47 | | 4,84E+06 | 6,68E+00 | | |
| | cpa | 24,99 | 24,075 | 7,39E+05 | 5,87E+00 | 6,14E+00 | |
| 21 | | 23,16 | | 2,63E+06 | 6,42E+00 | | |

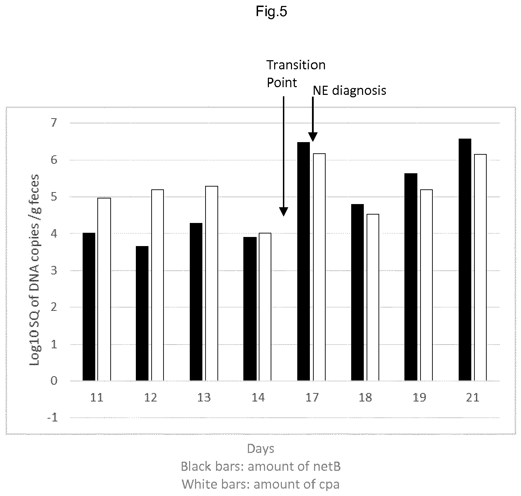

In this Example, the reversion of the ratio of the amounts of *netB* to *cpa* (Transition Point) occurred between day 14 and day 17. The outbreak of necrotic enteritis was established by veterinarian diagnosis (necropsy) on day 17.
Graphical presentation of the data of Example 1 may be found in Fig. 5.

<u>Summary</u>

The above experiments show that reversion of the relative amount of the marker genes *netB* and *cpa* occurs consistently prior to the pathological diagnosis of necrotic enteritis in an avian population. Said reversion of the ratio of the amounts of the marker genes *netB* to *cpa* thus qualifies as a diagnostic marker for predicting a near-term necrotic enteritis outbreak in an avian flock.

**Claims**

1. *In vitro* method for predicting a necrotic enteritis outbreak in an avian population, the method comprising:

   a) collecting fecal sample material deriving from the avian population at consecutive points in time; and
   b) determining the ratio of the amounts of the marker genes *netB* to *cpa,* contained in the sample material obtained in step a);

   wherein a reversion of the ratio of the amounts of *netB* to *cpa* over time predicts a near-term necrotic enteritis outbreak.

2. The method according to claim 1, wherein the fecal sample material of step a) is a composite fecal sample from randomly selected individual samples.

3. The method of claim 2, wherein the number of samples to be taken is determined using the following formula:

$$n_0 = \frac{Z^2 pq}{e^2}$$

   wherein

$n_0$ is the sample size recommendation

Z is 1.96 for 95% confidence level

p is the estimated portion of the population with the attribute in question q is 1-p, and

e is the confidence interval expressed as decimal.

4. The method of any one of claims 2 or 3, wherein the composite fecal sample is obtained by

(a1) dividing the animal house or the area in which the animal population is kept in a grid pattern of an equal number of uniform cells;

(a2) identifying at least one random sample collection site within the first cell and taking one first sample at said random sample collection site; and

(a3) sequentially collecting individual fecal samples in the remaining cells using the same relative sample collection sites within each cell;

and optionally

(a4) repeating steps (a2) and (a3) for at least one replicate sample.

5. The method according to any one of the preceding claims, wherein the avian population is a broiler flock.

6. The method according to claim 5, wherein the fecal sample material from the broiler flock is collected and analyzed on a daily basis starting from day 10.

7. The method according to any one of the preceding claims, wherein theratio of the amounts of the marker genes *netB* to *cpa* contained in the sample material obtained in step a) are determined via qPCR.

8. The method according to any one of the preceding claims, wherein one or more oligonucleotides selected from the group consisting of

a) oligonucleotides having a sequence identity of at least 80%, preferably at least 85, 90 or 95%, most preferably 100%, to the polynucleotide as depicted in SEQ ID NO:3;

b) oligonucleotides having a sequence identity of at least 80%, preferably at least 85, 90 or 95%, most preferably 100%, to the polynucleotide as depicted in SEQ ID NO: 4;

c) oligonucleotides having a sequence identity of at least 80%, preferably at least 85, 90 or 95%, most preferably 100%, to the polynucleotide as depicted in SEQ ID NO: 5;

d) oligonucleotides having a sequence identity of at least 80%, preferably at least 85, 90 or 95%, most preferably 100%, to the polynucleotide as depicted in SEQ ID NO: 6;

e) oligonucleotides having a sequence identity of at least 80%, preferably at least 85, 90 or 95%, most preferably 100%, to the polynucleotide as depicted in SEQ ID NO: 7;

f) oligonucleotides having a sequence identity of at least 80%, preferably at least 85, 90 or 95%, most preferably 100%, to the polynucleotide as depicted in SEQ ID NO:8;

g) oligonucleotides being complementary to the oligonucleotides according to (a) to (f);

h) oligonucleotides comprising any one of the oligonucleotides according to (a) to (g) and being elongated by not more than 5 base pairs compared to the oligonucleotides according to (a) to (g);

are used as a PCR primer and/or as a PCR probe.

9. Use of the method according to any one of claims 1 to 8 for the early detection of a necrotic enteritis outbreak.

10. *In vitro* method for controlling the necrotic enteritis status in an avian population, the method comprising monitoring the ratio of the amounts of the marker genes *netB* to *cpa* contained in fecal samples collected at consecutive points in time,

wherein

a) a reversion of the ratio of the amounts of *netB* to *cpa* over time indicates the necessity of a nutritional or therapeutic intervention, and

b) a re-reversion of the ratio of the amounts of *netB* to *cpa* over time after administering nutritional or therapeutic agents indicates the effectivity of the nutritional or therapeutic intervention.

11. The method according to claim 10, wherein the nutritional or therapeutic intervention involves administering substances selected from the group consisting of probiotic agents, prebiotic agents, botanicals, organic/fatty acids, bacteriophages and bacteriolytic enzymes or any combinations thereof.

12. The method according to any one of claims 10 and 11, wherein one or more oligonucleotides selected from the group consisting of

a) oligonucleotides having a sequence identity of at least 80%, preferably at least 85, 90 or 95%, most preferably 100%, to the polynucleotide as depicted in SEQ ID NO:3;
b) oligonucleotides having a sequence identity of at least 80%, preferably at least 85, 90 or 95%, most preferably 100%, to the polynucleotide as depicted in SEQ ID NO: 4;
c) oligonucleotides having a sequence identity of at least 80%, preferably at least 85, 90 or 95%, most preferably 100%, to the polynucleotide as depicted in SEQ ID NO: 5;
d) oligonucleotides having a sequence identity of at least 80%, preferably at least 85, 90 or 95%, most preferably 100%, to the polynucleotide as depicted in SEQ ID NO: 6;
e) oligonucleotides having a sequence identity of at least 80%, preferably at least 85, 90 or 95%, most preferably 100%, to the polynucleotide as depicted in SEQ ID NO: 7;
f) oligonucleotides having a sequence identity of at least 80%, preferably at least 85, 90 or 95%, most preferably 100%, to the polynucleotide as depicted in SEQ ID NO:8;
g) oligonucleotides being complementary to the oligonucleotides according to (a) to (f);
h) oligonucleotides comprising any one of the oligonucleotides according to (a) to (g) and being elongated by not more than 5 base pairs compared to the oligonucleotides according to (a) to (g);

are used as a PCR primer and/or as a PCR probe.

**Patentansprüche**

1. In-vitro-Verfahren zur Vorhersage eines Ausbruchs von nekrotischer Enteritis in einer Vogelpopulation; wobei das Verfahren Folgendes umfasst:

a) Sammeln von aus der Vogelpopulation stammendem Kotprobenmaterial an aufeinanderfolgenden Zeitpunkten; und
b) Bestimmen des Verhältnisses der Mengen der Markergene *netB* zu *cpa,* die im in Schritt a) erhaltenen Probenmaterial enthalten sind;

wobei eine Umkehr des Verhältnisses der Mengen von netB zu cpa im zeitlichen Verlauf einen zeitnahen Ausbruch von nekrotischer Enteritis vorhersagt.

2. Verfahren nach Anspruch 1, wobei es sich bei dem Kotprobenmaterial aus Schritt a) um eine Kotmischprobe aus willkürlich ausgewählten Einzelproben handelt.

3. Verfahren gemäß Anspruch 2, wobei die Zahl der zu nehmenden Proben unter Verwendung der folgenden Formel bestimmt wird:

$$n_0 = \frac{Z^2 pq}{e^2}$$

wobei

$n_0$ für die Probengrößenempfehlung steht,
Z gleich 1,96 bei einem Konfidenzniveau von 95% ist, p für den geschätzten Anteil der Population mit dem betreffenden Attribut steht,
q gleich 1-p ist und
e für das als Dezimalwert ausgedrückte Konfidenzintervall steht.

4. Verfahren gemäß einem der Ansprüche 2 oder 3, wobei die Kotmischprobe erhalten wird, indem

(a1) das Tierhaus oder die Fläche, auf der die Tierpopulation gehalten wird, rasterförmig in eine gleiche Anzahl einheitlicher Zellen aufgeteilt wird;

(a2) wenigstens eine willkürliche Probensammelstelle innerhalb der ersten Zelle identifiziert und eine erste Probe an der willkürlichen Probensammelstelle genommen wird; und

(a3) nacheinander Koteinzelproben in den restlichen Zellen unter Verwendung der gleichen jeweiligen Probensammelstellen innerhalb jeder Zelle gesammelt werden;

und gegebenenfalls

(a4) Schritte (a2) und (a3) für wenigstens eine Parallelprobe wiederholt werden.

5. Verfahren nach einem der vorhergehenden Ansprüche, wobei es sich bei der Vogelpopulation um Masthähnchen handelt.

6. Verfahren nach Anspruch 5, wobei das Kotprobenmaterial von den Masthähnchen von Tag 10 an täglich gesammelt und analysiert wird.

7. Verfahren nach einem der vorhergehenden Ansprüche, wobei das Verhältnis der Mengen der Markergene *netB* zu *cpa,* die im in Schritt a) erhaltenen Probenmaterial enthalten sind, über qPCR bestimmt wird.

8. Verfahren nach einem der vorhergehenden Ansprüche, wobei ein oder mehrere Oligonukleotide, die aus der Gruppe bestehend aus

a) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO:3;

b) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 4;

c) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 5;

d) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 6;

e) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 7;

f) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO:8;

g) Oligonukleotiden, die zu den Oligonukleotiden gemäß (a) bis (f) komplementär sind;

h) Oligonukleotiden, die eines der Oligonukleotide gemäß (a) bis (g) umfassen und verglichen mit den Oligonukleotiden gemäß (a) bis (g) um nicht mehr als 5 Basenpaare verlängert sind;

ausgewählt sind, als PCR-Primer und/oder als PCR-Sonde verwendet werden.

9. Verwendung des Verfahrens nach einem der Ansprüche 1 bis 8 zur Früherkennung eines Ausbruchs von nekrotischer Enteritis.

10. In-vitro-Verfahren zur Kontrolle des Nekrotische-Enteritis-Status in einer Vogelpopulation, wobei das Verfahren Überwachen des Verhältnisses der Mengen der Markergene *netB* zu *cpa,* die in an aufeinanderfolgenden Zeitpunkten gesammelten Kotproben enthalten sind, umfasst,

wobei

a) eine Umkehr des Verhältnisses der Mengen von *netB* zu *cpa* im zeitlichen Verlauf die Notwendigkeit eines nutritiven oder therapeutischen Eingriffs anzeigt und

b) eine erneute Umkehr des Verhältnisses der Mengen von *netB* zu *cpa* im zeitlichen Verlauf nach Verabreichen nutritiver bzw. therapeutischer Mittel die Wirksamkeit des nutritiven bzw. therapeutischen Eingriffs anzeigt.

11. Verfahren nach Anspruch 10, wobei der nutritive oder therapeutische Eingriff Verabreichen von Stoffen, die aus der Gruppe bestehend aus probiotischen Mitteln, präbiotischen Mitteln, pflanzlichen Stoffen, organischen Säuren/Fettsäuren, Bakteriophagen und bakteriolytischen Enzymen ausgewählt sind, oder beliebigen Kombinationen davon beinhaltet.

12. Verfahren nach einem der Ansprüche 10 und 11, wobei ein oder mehrere Oligonukleotide, die aus der Gruppe bestehend aus

a) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO:3;

b) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 4;

c) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 5;

d) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 6;

e) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO: 7;

f) Oligonukleotiden mit einer Sequenzidentität von wenigstens 80%, bevorzugt wenigstens 85, 90 oder 95%, am meisten bevorzugt 100%, mit dem Polynukleotid gemäß SEQ ID NO:8;

g) Oligonukleotiden, die zu den Oligonukleotiden gemäß (a) bis (f) komplementär sind;

h) Oligonukleotiden, die eines der Oligonukleotide gemäß (a) bis (g) umfassen und verglichen mit den Oligonukleotiden gemäß (a) bis (g) um nicht mehr als 5 Basenpaare verlängert sind;

ausgewählt sind, als PCR-Primer und/oder als PCR-Sonde verwendet werden.

## Revendications

1. Procédé *in vitro* pour prédire une épidémie d'entérite nécrotique dans une population aviaire, le procédé comprenant :

   a) la collecte d'un matériau d'échantillon fécal de la population aviaire à des moments consécutifs ; et
   b) la détermination du rapport des quantités des gènes marqueurs *netB* sur *cpa,* contenus dans le matériau d'échantillon, contenus dans le matériau d'échantillon obtenu dans l'étape a) ;

   une inversion du rapport des quantités de *netB* sur *cpa* dans le temps prédisant une épidémie d'entérite nécrotique à court terme.

2. Procédé selon la revendication 1, le matériau d'échantillon fécal de l'étape a) étant un matériau fécal composite d'échantillons individuels choisis de manière aléatoire.

3. Procédé selon la revendication 2, le nombre d'échantillons devant être pris étant déterminé en utilisant la formule suivante :

$$n_0 = \frac{Z^2 pq}{e^2}$$

   $n_0$ étant la recommandation de taille d'échantillon Z étant 1,96 pour un niveau de confiance de 95 % p étant la partie estimée de la population avec l'attribut en question q étant 1 - p, et
   e étant l'intervalle de confiance exprimé en décimale.

4. Procédé selon l'une quelconque des revendications 2 et 3, l'échantillon fécal composite étant obtenu par

   (a1) division du logement des animaux ou de la surface dans laquelle la population animale est gardée selon un motif en grille d'un nombre égal de cellules uniformes ;
   (a2) identification d'au moins un site de collecte d'échantillon aléatoire dans la première cellule et prise d'un premier échantillon au niveau dudit site de collecte d'échantillon aléatoire ; et
   (a3) collecte de manière séquentielle d'échantillons fécaux individuels dans les cellules restantes en utilisant les mêmes sites de collecte d'échantillon relatif dans chaque cellule ;
   et éventuellement
   (a4) répétition des étapes (a2) et (a3) pour au moins un échantillon répété.

**5.** Procédé selon l'une quelconque des revendications précédentes, la population aviaire étant un troupeau de poulets de chair.

**6.** Procédé selon la revendication 5, le matériau d'échantillon fécal du troupeau de poulets de chair étant collecté et analysé sur une base quotidienne débutant le jour en 10.

**7.** Procédé selon l'une quelconque des revendications précédentes, le rapport des quantités des gènes marqueurs *netB* sur *cpa* contenus dans le matériau d'échantillon obtenu dans l'étape a) étant déterminé via un qPCR.

**8.** Procédé selon l'une quelconque des revendications précédentes, un ou plusieurs oligonucléotides choisis dans le groupe constitué par

   a) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 3 ;
   b) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 4 ;
   c) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 5 ;
   d) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 6 ;
   e) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 7 ;
   f) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 8 ;
   g) des oligonucléotides qui sont complémentaires aux oligonucléotides selon (a) à (f) ;
   h) des oligonucléotides comprenant l'un quelconque parmi les oligonucléotides selon (a) à (g) et qui sont allongés de pas plus de 5 paires de base par comparaison avec les oligonucléotides selon (a) à (g) ;

étant utilisés comme une amorce de PCR et/ou comme une sonde de PCR.

**9.** Utilisation du procédé selon l'une quelconque des revendications 1 à 8 pour la détection précoce d'une épidémie d'entérite nécrotique.

**10.** Procédé *in vitro* pour la régulation de l'état d'entérite nécrotique dans une population aviaire, le procédé comprenant la surveillance du rapport des gènes marqueurs *netB* sur *cpa* contenus dans des échantillons fécaux collectés à des points consécutifs dans le temps,

   a) une inversion du rapport des quantités de *netB* sur *cpa* dans le temps indiquant la nécessité d'une intervention nutritionnelle ou thérapeutique, et
   b) une ré-inversion du rapport des quantités de *netB* sur *cpa* dans le temps après administration d'agents nutritionnels ou thérapeutiques indiquant l'efficacité de l'intervention nutritionnelle ou thérapeutique.

**11.** Procédé selon la revendication 10, l'intervention nutritionnelle ou thérapeutique impliquant une administration de substances choisies dans le groupe constitué par des agents probiotiques, des agents prébiotiques, des plantes, des acides organiques/gras, des bactériophages et des enzymes bactériolytiques ou de quelconques combinaisons correspondantes.

**12.** Procédé selon l'une quelconque des revendications 10 et 11, un ou plusieurs oligonucléotides choisis dans le groupe constitué par

   a) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 3 ;
   b) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 4 ;
   c) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 5 ;
   d) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 6 ;

e) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 7 ;

f) des oligonucléotides ayant une identité de séquence d'au moins 80 %, préférablement d'au moins 85, 90 ou 95 %, le plus préférablement 100 %, avec le polynucléotide tel que décrit dans la SEQ ID NO: 8 ;

g) des oligonucléotides qui sont complémentaires aux oligonucléotides selon (a) à (f) ;

h) des oligonucléotides comprenant l'un quelconque parmi les oligonucléotides selon (a) à (g) et qui sont allongés de pas plus de 5 paires de base par comparaison avec les oligonucléotides selon (a) à (g) ;

étant utilisés comme une amorce de PCR et/ou comme une sonde de PCR.

Fig. 1

Fig. 2

Fig. 3

Black bars: amount of netB
White bars: amount of cpa

Fig. 4

Black bars: amount of netB
White bars: amount of cpa

Fig.5

Transition Point — NE diagnosis

Black bars: amount of netB
White bars: amount of cpa

## REFERENCES CITED IN THE DESCRIPTION

### Patent documents cited in the description

- WO 2016142146 A

### Non-patent literature cited in the description

- **ROOD, J. I.** Virulence genes of Clostridium perfringens. *Annual Review of Microbiology,* 1998, vol. 52, 333-360
- **POPOFF, M. R. ; P. BOUVET.** Genetic characteristics of toxigenic Clostridia and toxin gene evolution. *Toxicon,* 2013, vol. 75, 63-89
- **WADE, B. ; KEYBURN, A.L.** The true cost of necrotic enteritis. *World Poultry,* 2015, vol. 31, 16-17
- **KEYBURN, A. L. et al.** Alpha-toxin of Clostridium perfringens is not an essential virulence factor in necrotic enteritis in chickens. *Infection and Immunity,* 2006, vol. 74 (11), 6496-6500
- **TITBALL, R. W. et al.** The Clostridium perfringens α-toxin. *Anaerobe,* 1999, vol. 5 (2), 51-64
- **KEYBURN, A. L. et al.** NetS, a new toxin that is associated with avian necrotic enteritis caused by Clostridium perfringens. *PLoS Pathogens,* 2008, vol. 4 (2
- **TIMBERMONT, L. et al.** Necrotic enteritis in broilers: An updated review on the pathogenesis. *Avian Pathology,* 2011, vol. 40 (4), 341-347
- **SHOJADOOST, B. ; VINCE, A.R. ; PRESCOTT, J.F.** The successful experimental induction of necrotic enteritis in chickens by Clostridium perfringens: a critical review. *Vet. Res.,* 2012, vol. 43, 74
- **FERNANDES DA COSTA, S.P. ; MOT, D. ; BOKORI-BROWN, M. ; SAVVA, C.G. ; BASAK, A.K. ; VAN IMMERSEEL, F. ; TITBALL, R.W.** Protection against avian necrotic enteritis after immunisation with NetB genetic or formaldehyde toxoids. *Vaccine,* 2013, vol. 31, 4003-4008
- **WILLIAMS, R.B. ; MARSHALL, R.N. ; LA RAGIONE, R.M. ; CATCHPOLE, J.** A new method for the experimental production of necrotic enteritis and its use for studies on the relationships between necrotic enteritis, coccidiosis and anticoccidial vaccination of chickens. *Parasitol. Res.,* 2003, vol. 90, 19-26
- **WU, S.B. ; RODGERS, N. ; CHOCT, M.** Optimized necrotic enteritis model producing clinical and subclinical infection of Clostridium perfringens in broiler chickens. *Avian Dis.,* 2010, vol. 54, 1058-1065
- **WU S-B ; STANLEY D ; RODGERS N ; SWICK RA ; MOORE RJ.** Two necrotic enteritis predisposing factors, dietary fishmeal and eimeria infection, induce large changes in the caecal microbiota of broiler chickens. *Vet Microbiol,* 2014, vol. 169, 188e97